# EUROPEAN PATENT SPECIFICATION

(11) **EP 3 855 186 B1**
(45) Date of publication and mention of the grant of the patent: **06.12.2023**
(21) Application number: 21173934.7
(22) Date of filing: 14.05.2021
(51) Int. Cl.: G01N 33/68

(54) **A METHOD FOR DETERMINING THE EFFICACY OF A SARS-COV-2 VACCINE**
VERFAHREN ZUR BESTIMMUNG DER WIRKSAMKEIT EINES IMPFSTOFFS
PROCÉDÉ PERMETTANT DE DÉTERMINER L'EFFICACITÉ D'UN VACCIN

(30) Priority: 15.05.2020 EP 20175031
(43) Date of publication of application: 28.07.2021
(73) Proprietor: Euroimmun Medizinische Labordiagnostika AG, 23560 Lübeck (DE)
(72) Inventor: Stöcker, Winfried, 23627, Gross Grönau (DE); Steinhagen, Katja, 23627, Gross Grönau (DE); Komorowski, Lars, 23909, Ratzeburg (DE)

(56) References cited:
- CN-A- 111 088 283
- Nisreen M.A. Okba ET AL: "SARS-CoV-2 specific antibody responses in COVID-19 patients", medRxiv, 20 March 2020 (2020-03-20), XP055727454, DOI: 10.1101/2020.03.18.20038059 Retrieved from the Internet: URL:https://www.medrxiv.org/content/10.110 1/2020.03.18.20038059v1.full.pdf
- TO KELVIN KAI-WANG ET AL: "Temporal profiles of viral load in posterior oropharyngeal saliva samples and serum antibody responses during infection by SARS-CoV-2: an observational cohort study", THE LANCET INFECTIOUS DISEASES, ELSEVIER, AMSTERDAM, NL, vol. 20, no. 5, 23 March 2020 (2020-03-23) , pages 565-574, XP086152240, ISSN: 1473-3099, DOI: 10.1016/S1473-3099(20)30196-1 [retrieved on 2020-03-23]
- Ebi: "EBI database entry for QIU80910", , 21 April 2020 (2020-04-21), XP055729000, Retrieved from the Internet: URL:https://www.ebi.ac.uk/ena/browser/api/ embl/QIU80910.1?lineLimit=1000 [retrieved on 2020-09-09]

## Description

The invention relates to a method for distinguishing an immunized healthy subject, which is a vaccinated subject, from a non-immunized healthy subject comprising the step determining in a blood sample the presence or absence of an IgA class antibody to SEQ ID NO1, wherein the sample comprises a set of representative antibodies from the subject, and wherein the blood sample is from a human subject, a method for method for testing the efficacy of a vaccine, comprising the step determining in a blood sample the presence or absence of an IgA class antibody to SEQ ID NO1, wherein the sample comprises a set of representative antibodies from the subject, and wherein the blood sample is from a human subject and a use of a kit for distinguishing an immunized subject, which is a vaccinated subject, from a non-immunized subject, wherein the kit comprises a carrier coated with a polypeptide comprising SEQ ID NO1 or a variant thereof and a means for detecting a specifically captured antibody, comprising the step determining in a blood sample the presence or absence of an IgA class antibody to SEQ ID NO1, wherein the sample comprises a set of representative antibodies from the subject.

At the end of 2019, a rising number of pneumonia patients with unknown pathogen emerged from Wuhan, the capital of Hubei province, China, to nearly the entirety of China. A novel coronavirus was isolated and based on its phylogeny, taxonomy and established practice, the Coronavirus Study Group (CSG) recognized it as a sister to severe acute respiratory syndrome coronavirus (SARS-CoV or SARS-CoV-1) and labeled it as severe acute respiratory syndrome coronavirus 2 (SARS-CoV-2). Although SARS-CoV-2 is generally less pathogenic than SARS-CoV and Middle East respiratory syndrome coronavirus (MERS-CoV), it has a relatively high transmissibility. Since symptoms may be mild and may be confused with a cold, there is the danger that patients are unaware that they have been infected and may help the virus spread further.

SARS-CoV-2 is a coronavirus which has four major structural proteins, specifically the spike (S), envelope (E), membrane (M) and nucleocapsid (N) proteins. The N protein holds the RNA genome, while the other three structural proteins are components of the viral envelope. The S protein is responsible for allowing the virus to attach and fuse to the membrane of a host cell. It comprises an S1 domain which mediates the attachment and an S2 domain which mediates the fusion of the viral cellular membrane with the host cell. The S1 domain comprises the receptor binding domain (RBD), the binding site to the receptor angiotensin converting enzyme 2 (ACE2) on human host cells. It is now clear that the RBD is the major binding site of neutralizing antibodies which block the interaction between the virus and its host cells, thus conferring immunity. By contrast to SARS-CoV-1 and SARS-CoV-2, which are associated with a high mortality and severe illness, other coronaviruses exist which are associated with a mild and passing illness, such as coronaviruses 229E, NL63, OC43 and HKU1. These coronaviruses are frequently associated with common cold, in particular among children.

In view of the massive impact of SARS-CoV-2 on economy and healthcare systems, there is considerable interest in being able to distinguish immunized vaccinated healthy subjects from non-immunized healthy and infected subjects. Vaccinated immunized subjects may be able to return to regular work sooner than non-immunized patients.

Vaccinated patients may be interested in being able to or may in fact be required to demonstrate their immunization status, more specifically the fact that they have been vaccinated. This may be required if the vaccination is insufficiently documented or was performed in a country with sub-standard health standards or the quality of the vaccine used is questioned. In addition, mistakes, for example the use of physiological salt buffers rather than the vaccine, have been reported. Not in the least, falsified documents confirming vaccination are circulating.

Therefore, confirmation that the vaccination has been effective and has been carried out may be required before the person is allowed to return to work, for example in an office shared with others, or to attend social or public events such as concerts or even immigrate from countries with a high number of active SARS-CoV-2 cases.

Most commercially available methods for determining the immune states relate to the detection of IgG class antibodies, because it is generally the most persistent reaction after an immunization to a viral or bacterial pathogen and may last for months or even years. However, IgG class antibodies may be detectable only after approximately 20 days (Meyer, Drosten & Müller, Virus Research 194 (2014); 175-186). This means that serological confirmation of a successful vaccination is available only late after the vaccination. Vaccinated persons may be excluded from coming to work or joining social or public events meanwhile.

Various projects aim to develop vaccines, based on a variety of technologies, including adenovirus vectors, RNA, inactivated SARS-CoV-2 virus and isolated SARS-CoV-2 components, in particular Nucleocapsid protein and S1. As of February 2021, more than 200 candidate vaccines were being developed, and at least five of them were tested in clinical trials. All of these candidates generate immunity against the S protein (Mclntyre P, Joo YJ, Chiu C, Flana-gan K, Macartney K. COVID-19 vaccines - are we there yet?. Aust Prescr. 2021;44(1):19-25). If such vaccines are to be developed, it is important to be able to test their efficacy, for example in clinical trials on humans.

WO2005118813 discloses the detection of IgA class antibodies to SARS-CoV-1 in fecal and mucosal samples from mice following the administration of a vaccine based on a spike protein. There is no indication that IgA could be detected in serum, let alone in humans, let alone more rapidly than IgG after a vaccination or in fact early after the vaccination at all.

Okba et al. Emerg Infect Dis. 2020 Jul;26(7):1478-1488., first published in March 2021 in a non peer-reviewed format on medRxiv (doi 10.1101/2020.03.18.20038059), disclose various assays for the detection of antibodies in blood samples from patients suffering from a SARS-CoV-2 infection (but not from vaccinated patients), including IgA class antibodies. It is not disclosed that IgA class antibodies emerge before IgG class antibodies. The use of an assay to determine whether a subject has been vaccinated is not disclosed.

To et al. (The Lancet Infectious diseases 20 (5) 565-574) discloses measuring antibody levels against SARS-CoV-2 nucleoprotein and surface spike protein receptor binding domain (RBD) in patients with a SARS-CoV-2 infection.

CN111088283 discloses experiments for determining the most efficient vaccination route in a mouse based on IgA detection.

Therefore, the problem underlying the present invention is to provide a method for such a distinction, in particular where a subject may have been vaccinated by administration of a composition comprising the S1 protein from SARS-CoV-2 or not.

Another problem underlying the present invention is to provide a method for distinguishing non-immunized healthy, immunized health and infected subjects.

Another problem underlying the present invention is to provide one of the aforementioned methods, whereas an initial result may be obtained as soon as possible, in particular before the IgG class antibody response is detectable.

In a first aspect, the problem is solved by a method for distinguishing an immunized healthy subject, which is a vaccinated subject, from a non-immunized healthy subject comprising the step determining in a blood sample the presence or absence of an IgA class antibody to SEQ ID NO1, wherein the sample comprises a set of representative antibodies from the subject, and wherein the blood sample is from a human subject.

In a second aspect, the problem is solved by a method for testing the efficacy of a vaccine, comprising the step determining in a blood sample the presence or absence of an IgA class antibody to SEQ ID NO1, wherein the sample comprises a set of representative antibodies from the subject, and wherein the blood sample is from a human subject.

In a third aspect, the problem is solved by a use of a kit for distinguishing an immunized subject, which is a vaccinated subject, from a non-immunized subject, wherein the kit comprises a carrier coated with a polypeptide comprising SEQ ID NO1 or a variant thereof and a means for detecting a specifically captured antibody
wherein
a. the polypeptide comprises the full length-sequence of SEQ ID NO1 or a variant of SEQ ID NO1, which has a sequence identity to SEQ ID NO1 of at least 80, 85, 90, 92, 94,95, 96, 97, 98, 99 %, optionally fused to one or more artificial linkers, affinity tags and/or other antigens, or
b. the variant is a truncated SEQ ID NO1, i. e. less than the full-length sequence of SEQ ID NO1, wherein the truncation is at the N-terminus, C-terminus or both, optionally fused to one or more artificial linkers and/or affinity tags, and
   i. comprises a fragment of SEQ ID NO1 comprising at least 25, 50, 75, 100, 150, 200, 250, 300, 400, 500 or 600 successive amino acids of SEQ ID NO1 and/or
   ii. comprises a fragment of SEQ ID NO1 comprising at least 200 successive amino acids with a sequence identity to the reference fragment of SEQ ID NO1 of at least 80, 85, 90, 92, 94,95, 96, 97, 98, or 99 %,
      and wherein the variant has the ability to bind to an antibody to SEQ ID NO1 from a sample from a patient suffering from a SARS-CoV-2 infection,
   comprising the step determining in a blood sample the presence or absence of an IgA class antibody to SEQ ID NO1, wherein the sample comprises a set of representative antibodies from the subject.

In a preferred embodiment, a non-immunized healthy, an immunized healthy and an infected subject is distinguished.

In a preferred embodiment, a carrier coated with a polypeptide comprising SEQ ID NO1 or a variant thereof is contacted with the sample under conditions allowing for binding of any antibodies to SEQ ID NO1, and a secondary antibody or means binding to the IgA class antibody and carrying a detectable label is contacted with the carrier under conditions allowing formation of a complex between any bound antibody and the secondary antibody or means,
wherein
a. the polypeptide comprises the full length-sequence of SEQ ID NO1 or a variant of SEQ ID NO1, which has a sequence identity to SEQ ID NO1 of at least 80, 85, 90, 92, 94,95, 96, 97, 98, 99 %, optionally fused to one or more artificial linkers, affinity tags and/or other antigens, or
b. the variant is a truncated SEQ ID NO1, i. e. less than the full-length sequence of SEQ ID NO1, wherein the truncation is at the N-terminus, C-terminus or both, optionally fused to one or more artificial linkers and/or affinity tags, and
   i. comprises a fragment of SEQ ID NO1 comprising at least 25, 50, 75, 100, 150, 200, 250, 300, 400, 500 or 600 successive amino acids of SEQ ID NO1 and/or
   ii. comprises a fragment of SEQ ID NO1 comprising at least 200 successive amino acids with a sequence identity to the reference fragment of SEQ ID NO1 of at least 80, 85, 90, 92, 94,95, 96, 97, 98, or 99 %,
      and wherein the variant has the ability to bind to an antibody to SEQ ID NO1 from a sample from a patient suffering from a SARS-CoV-2 infection.

In a preferred embodiment, the carrier is selected from the group comprising a bead, a test strip, a microtiter plate, a microarray, a blot, preferably from the group comprising western blot, line blot and dot blot, a glass surface, a slide, a biochip and a membrane, and is most preferably a microtiter plate or a line blot.

In a preferred embodiment, an IgA class antibody to SEQ ID NO1 is detected before the emergence of IgG class antibodies to SEQ ID NO1.

In a preferred embodiment, the presence or absence of an antibody from the group of classes comprising IgG, IgA and IgM to SEQ ID NO1 is detected.

In a preferred embodiment, the presence or absence of an antibody to SEQ ID NO1 is monitored for at least 1, 2, 3 or 4 weeks.

In a preferred embodiment, a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminescence immunoassays and immunofluorescence techniques is used to detect the antibody.

In a preferred embodiment, a method from the group comprising a competitive assay, a capture bridge assay, an immunometric assay, a class-specific second antibody on the solid phase, a class capture assay and an ELISA is used to detect the antibody.

In a preferred embodiment, the sample is a blood sample from the group comprising serum, plasma and whole blood.

In a preferred embodiment, a vaccination may be recognized by the production of a detectable level of antibodies.

In a preferred embodiment, the detectable level of antibodies comprises IgA, IgM and IgG class antibodies to SEQ ID NO1.

In a preferred embodiment, two or three or more shots of vaccine were administered to the subject.

In a preferred embodiment, a means binding to the IgA class antibody is used which is (a) a secondary antibody binding to human IgA class antibodies or (b) a labeled polypeptide which comprises SEQ ID NO1 or a variant thereof which binds to an antibody from the group of classes comprising IgG, IgA and IgM to SEQ ID NO1,
wherein
a. the polypeptide comprises the full length-sequence of SEQ ID NO1 or a variant of SEQ ID NO1, which has a sequence identity to SEQ ID NO1 of at least 80, 85, 90, 92, 94,95, 96, 97, 98, 99 %, optionally fused to one or more artificial linkers, affinity tags and/or other antigens, or
b. the variant is a truncated SEQ ID NO1, i. e. less than the full-length sequence of SEQ ID NO1, wherein the truncation is at the N-terminus, C-terminus or both, optionally fused to one or more artificial linkers and/or affinity tags, and
   i. comprises a fragment of SEQ ID NO1 comprising at least 25, 50, 75, 100, 150, 200, 250, 300, 400, 500 or 600 successive amino acids of SEQ ID NO1 and/or
   ii. comprises a fragment of SEQ ID NO1 comprising at least 200 successive amino acids with a sequence identity to the reference fragment of SEQ ID NO1 of at least 80, 85, 90, 92, 94,95, 96, 97, 98, or 99 %,
and wherein the variant has the ability to bind to an antibody to SEQ ID NO1 from a sample from a patient suffering from a SARS-CoV-2

In a preferred embodiment,, the problem is solved by a method for determining the immune status of a subject, more specifically whether the subject has been vaccinated with a composition comprising SEQ ID NO1 or a variant thereof which composition triggers the production of antibodies in the subject or had no previous exposure to SARS-CoV-2 or any component of it, comprising the step determining in a sample the presence or absence of an IgA class antibody to SEQ ID NO1, wherein the sample comprises a set of representative antibodies from the subject.

In a preferred embodiment,, the problem underlying the present invention is solved by a method for determining whether a subject produces antibodies as a result of administration of a vaccine, comprising the step detecting in a sample comprising antibodies from said subject
a) the absence or presence of an antibody to SEQ ID NO1 and
b) the absence or presence of an antibody to SEQ DI NO2.

In a preferred embodiment, the problem underlying the present invention is solved by a method for distinguishing whether a subject produces antibodies as a result of administration of a vaccine or as a result of a previous corona virus infection, preferably SARS-CoV-2 infection comprising the step detecting in a sample comprising antibodies from said subject
a) the absence or presence of an antibody to SEQ ID NO1 and
b) the absence or presence of an antibody to SEQ DI NO2.

In a preferred embodiment, the antibody to SEQ ID NO2 is an antibody to SEQ ID NO3.

In a preferred embodiment, the antibody to SEQ ID NO1 is an IgA, IgG or IgM class antibody, preferably IgG or IgM, more preferably IgG.

In a preferred antibody, the antibody to SEQ ID NO2 is an IgA, IgG or IgM class antibody, preferably IgM or IgG, more preferably IgG.

In a preferred embodiment, the presence or absence of the antibody in step a) is monitored over at least one, preferably two, more preferably four weeks.

In a preferred embodiment,, the problem underlying the present invention is solved by use of a first polypeptide comprising SEQ ID NO1 or a variant thereof and a second polypeptide comprising SEQ ID NO2 or a variant thereof for determining whether a subject produces antibodies as a result of administration of a vaccine.

In a preferred embodiment, the carrier is selected from the group comprising a bead, a test strip, a microtiter plate, a microarray, a blot, preferably from the group comprising western blot, line blot and dot blot, a glass surface, a slide, a biochip and a membrane, and is most preferably a microtiter plate or a line blot.

In a preferred embodiment, the method is for determining the efficacy of a vaccine candidate comprising a polypeptide comprising SEQ ID NO1 or a variant thereof.

In a preferred embodiment, the vaccine does not comprise SEQ ID NO2 or an immunogenic variant thereof, preferably an immunogenic variant.

In a preferred embodiment, it is monitored whether an antibody to SEQ ID NO1 (S1) is present, preferably for at least one, two, three, four, five or six weeks, preferably four weeks.

In a preferred embodiment, it is monitored whether an antibody to SEQ ID NO2 is present, preferably for at least one, two, three, four, five or six weeks, preferably four weeks.

In a preferred embodiment, the sample is a blood sample, preferably from the group comprising serum, plasma and whole blood.

In a preferred embodiment, the methods and the kit according to the invention are used to increase the sensitivity of a method for detecting the immunization status of a subject, more specifically whether the subject has been vaccinated with a composition comprising SEQ ID NO1 comprising the step detecting in a sample comprising antibodies from said subject. Preferably the method or use or reagent is used no longer than 28, 21, 14, 12, 10, 8, 7, 6, 5 or 4 days after the first administration of the vaccine.

The present invention centers around determining the immune status of a subject, more specifically whether the subject has been infected previously, but is now healthy, has been vaccinated or had no previous exposure to SARS-CoV-2 or any component of it. It is based on the inventors' surprising finding that following a vaccination with a vaccine comprising SEQ ID NO1 or a fragment thereof, IgA class antibodies to SEQ ID NO1 may be detectable before IgG class antibodies to SEQ ID NO1 are. In other words, detecting IgA class antibodies increases the chance to confirm a successful vaccination earlier than using a conventional assay based on the detection IgG class antibodies.

This finding of the inventors was subsequently confirmed by independent scientists in peer published papers (e.g. Sterlin et al., IgA dominates the early neutralizing antibody response to SARS-CoV-2, Science Translational Medicine, 2021 Vol. 13, Issue 577). IgA antibodies are generally not considered diagnostically relevant by the person skilled in the art except for specific cases, such as in cases of infections by entero viruses (Gressner/Arndt, Lexikon der Medizinischen Labordiagnostik, 2. Auflage, Springer, 2013, page 1387), but if they appear, their titer will typically be lower than IgM and IgG class antibodies, and they may appear later. According to publications relating to the diagnosis of emerging corona viruses such as SARS-CoV, IgA class antibodies do not appear earlier than IgG after infections (Meyer, Drosten & Müller, Virus Research 194 (2014); 175-186). As far as SARS-CoV-2 is concerned, a high sensitivity of diagnostic assays detecting IgA class antibodies to SEQ ID NO1 has been reported, but IgA and IgG class antibodies first appeared at the same time, in a sample taken after approximately 12 days.

In a preferred embodiment, the term "SARS-CoV-2", as used herein, refers to a virus characterized by the genome deposited on GenBank under accession code MN908947 or SEQ ID NO: 13, preferably as shown in SEQ ID NO: 13, and derivatives thereof having at least 80, preferably 85, preferably 88, preferably 90, preferably 91, preferably 92, preferably 93, preferably 94, preferably 95, preferably 96, preferably 97, preferably 98, preferably 99, preferably 99.5, preferably 99.8, preferably 99.9 or 99.99 percent sequence identity over the entire genome nucleotide sequence. More preferably, mutants such as those from the group comprising the U.K. variant B.1.1.7, the South African variant B.1.351, the Brazilian variant P.1 and the Mink Variant from Denmark are included.

In a preferred embodiment, the SARS-CoV-2 infection relating to a vaccination may be associated with the U.K. variant B.1.1.7 characterized by a spike protein having, with reference to SEQ ID NO1, one or more mutations, preferably all mutations from the group comprising deletion(s) in His 54 and/or Val55, Gln486Tyr, Ala555Asp, Asp599Gly and Pro666His. Preferably, a variant of SEQ ID NO1 having one or more mutations, preferably all from the group comprising deletion(s) in His 54 and/or Val55, Gln486Tyr, Ala555Asp, Asp599Gly and Pro666His, is used for reagents, methods and uses according to the present invention. A variant of SEQ ID NO1 comprising all these mutations is represented by SEQ ID NO: 51.

In a preferred embodiment, the SARS-CoV-2 infection relating to a vaccination may be associated with the South African variant B.1.351 characterized by a spike protein having, with reference to SEQ ID NO1, one or more mutations, preferably all mutations from the group comprising Lys402Gln, Glu469Lys, Gln486Tyr and Asp599Gly. Preferably, a variant of SEQ ID NO1 having one or more mutations, preferably all from the group comprising Lys402Gln, Glu469Lys, Gln486Tyr and Asp599Gly, is used for reagents, methods and uses according to the present invention. A variant of SEQ ID NO1 comprising all these mutations is represented by SEQ ID NO: 52.

In a preferred embodiment, the SARS-CoV-2 infection relating to a vaccination may be associated with the Brazilian variant P.1 characterized by a spike protein having, with reference to SEQ ID NO1, one or more mutations, preferably all mutations from the group comprising Glu469Lys and Gln486Tyr. Preferably, a variant of SEQ ID NO1 having one or more mutations, preferably all from the group comprising Glu469Lys and Gln486Tyr, is used for reagents, methods and uses according to the present invention. A variant of SEQ ID NO1 comprising all these mutations is represented by SEQ ID NO: 53.

In a preferred embodiment, the SARS-CoV-2 infection relating to a vaccination may be associated with the Mink Variant from Denmark characterized by a spike protein having, with reference to SEQ ID NO1, one or more mutations, preferably all mutations from the group comprising deletion(s) in His 54 and/or Val55, Asp599Gly and Tyr438Phe. Preferably, a variant of SEQ ID NO1 having one or more mutations, preferably all from the group comprising deletion(s) in His 54 and/or Val55, Asp599Gly and Tyr438Phe, is used for reagents, methods and uses according to the present invention. A variant of SEQ ID NO1 comprising all these mutations is represented by SEQ ID NO: 54.

The sample is from a subject who has been or claims to have been vaccinated using a commercially vaccine based on SEQ ID NO1 or a variant thereof such as COVID-19 Moderna^{®}. It may be obtained from a subject from an anonymous cohort of subjects for the purpose of research, for example to study the proportion of vaccinated subjects in a population or in a subpopulation, for example a group which has been vaccinated. It may be obtained from a human subject in a clinical trial, where the titers of antibodies are preferably monitored. In a preferred embodiment, it comprises a set of representative antibodies from the subject. In another preferred embodiment, it comprises a fraction of the sample enriched with regard to antibodies, preferably with regard to a class of antibodies, for example IgG, IgA and IgM. The sample is a blood sample, preferably selected from the group comprising whole blood, serum or plasma.

In a preferred embodiment, the presence or absence of an antibody to SEQ ID NO1 is determined, which is an antibody from the group of classes comprising IgG, IgA and IgM. The class of the antibody to SEQ ID NO1 may be IgA.

In a preferred embodiment, the presence or absence of an antibody to SEQ ID NO2 is determined, preferably an antibody from the group of classes comprising IgG, IgA and IgM. The class of the antibody to SEQ ID NO2 may be IgG. The class of the antibody to SEQ ID NO2 may be IgM. The class of the antibody to SEQ ID NO2 may be IgA. In a more preferred embodiment, the presence or absence of an antibody to SEQ ID NO3 is determined, preferably an antibody from the group of classes comprising IgG, IgA and IgM. The class of the antibody to SEQ ID NO3 may be IgG. The class of the antibody to SEQ ID NO3 may be IgM. The class of the antibody to SEQ ID NO3 may be IgA.

Preferably antibodies of the same class against SEQ ID NO1 and SEQ ID NO2 are detected, preferably IgA, IgM or IgG, more preferably IgG. In a preferred embodiment, the inventive method is carried out in two steps: in a first step, IgG class antibodies to SEQ ID NO1 are detected and if the result is negative, IgA class antibodies, optionally in addition IgM class antibodies, to SEQ ID NO1 are detected to enhance the overall sensitivity.

In a preferred embodiment, the methods and use of the present invention are configured such that the presence or absence of an antibody to SEQ ID NO: 1 can be detected, without determining whether the antibody detected belongs to a certain immunoglobulin class. This is often referred to as detecting the "total antibodies" to one or more antigens such as SEQ ID NO: 1. Detecting total antibodies, including at least IgA and IgG and preferably in addition IgM, increases the sensitivity of the assay, since IgA class antibodies are detected as part of the total antibodies to SEQ ID NO: 1. It should be mentioned that the time point of the vaccination may be unknown, in particular if a sample from an anonymous subject is examined or if a person with memory loss, a symptom of many neurological diseases or associated with age, is checked. In this case, the range of possible samples, in particular in a large cohort, may comprise samples from recently vaccinated persons in which antibodies can be detected only if the presence of absence of IgA class antibodies to SEQ ID NO1 is detected. Hence, the assay according to the present invention will increase the overall sensitivity.

Preferably, an antibody to SEQ ID NO: 1 can be distinguished from an antibody to another SARS-CoV-2 antigen such as N protein (SEQ ID NO: 30) or S2 domain (SEQ ID NO: 32), more preferably because other SARS-CoV-2 antigens are absent or spatially separated or an antibody to such another SARS-CoV-2 produces a signal which can be distinguished from a signal produced by an antibody to SEQ ID NO: 1.

In a preferred embodiment, the methods and use of the present invention are configured such that presence or absence of an antibody to SEQ ID NO: 1 can be detected, without determining whether the antibody detected binds to SEQ ID NO: 1 or to another SARS-CoV-2 antigen such as N protein or S2 protein. This may be accomplished by using a mixture of antigens comprising SEQ ID NO: 1 or a variant thereof in combination with N protein (SEQ ID NO: 30) or a variant thereof or S2 domain (SEQ ID NO: 32) or a variant thereof. In a more preferred embodiment, the whole spike protein comprising S1 and S2 domain (SEQ ID NO: 32) may be used, optionally in combination with N protein (SEQ ID NO: 30). Again, the sensitivity of such an assay is increased since IgA class antibodies are detected as part of the total antibodies to SEQ ID NO: 1.

In a preferred embodiment, the products, methods and uses of the present invention are configured such that presence or absence of an antibody to SEQ ID NO: 1 can be detected, without determining whether the antibody detected belongs to a certain immunoglobulin class. This procedure is often referred to as detecting the "total antibodies" to one or more antigens such as SEQ ID NO: 1.

If an antibody to SEQ ID NO1 is present in the sample, this indicates that the subject has been vaccinated, suffers from a SARS-CoV-2 infection or suffered from a SARS-CoV-2 infection. In a preferred embodiment, detection of an IgA class antibody to SEQ ID NO1 indicates a recent infection or recent vaccination. In a preferred embodiment, additional parameters such as whether SARS-CoV-2 infection symptoms or additional antibodies are detectable or whether or not the subject has likely been exposed to infected patients recently may be considered to distinguish between an infection and a vaccination. For example, if a subject has recently been vaccinated, preferably at least 3, 4, 5, 6 days, 1, 2, 3, 4, 6, 8 or 10 weeks before the sample was taken, an IgA class antibody indicates an increased likelihood that the vaccination was effective, especially if the patient resided in an area with a low incidence of SARS-CoV-2 infections. Personal behavior such as quarantine or strict social distancing may also be considered. If an antibody to SEQ ID NO1 is present in the sample and optionally an antibody to SEQ DI NO2 is absent, this indicates an increased likelihood that the subject has been successfully vaccinated with a vaccine comprising S1 protein from SARS-CoV-2 or a variant thereof, which preferably does not comprise a polypeptide comprising SEQ ID NO2 or a variant thereof. Likewise, If an antibody to SEQ ID NO2 is present in the sample and an antibody to SEQ DI NO1 is absent, this indicates an increased likelihood that the subject has been successfully vaccinated with a vaccine comprising nucleocapsid protein from SARS-CoV-2 or a variant thereof, which may comprise a polypeptide comprising SEQ ID NO2, preferably SEQ ID NO3 or a variant thereof.

Alternatively, if the level of antibodies to SEQ ID NO1 increases, while the level of antibodies to SEQ ID NO2 does not increase, this indicates an increased likelihood that the subject has been successfully vaccinated with a vaccine comprising SEQ ID NO1 or a variant thereof rather than having been exposed to full SARS-CoV-2, for example by way of an infection.

If both an antibody to SEQ ID NO1 and an antibody to SEQ ID NO2 are detectable or their levels increase, this suggests that the patient is or was infected with SARS-CoV-2. If the patient has symptoms and/or an IgM and/or IgA class antibodies can be detected or if the levels of IgM and/or IgA and/or IgG class antibodies are increasing, this suggests an active infection. The absence of symptoms and/or IgM and/or IgA class antibodies suggests a past infection. A PCR-based diagnostic assay for SARS-CoV-2 may be run in addition, in particular if a very early stage of the infection is suspected.

In a preferred embodiment, the presence or absence of symptoms typical for SARS-CoV and SARS-CoV-2 are taken into account to distinguish whether the subject suffers or has suffered from SARS or SARS-CoV2.

In a preferred embodiment, the presence or absence of an antibody to SEQ ID NO1 is detected, preferably monitored for at least 1, 2, 3, 4, 6, 8, 10, 12 or 16 weeks. The presence of an antibody to SEQ ID NO2, preferably SEQ ID NO3 may be determined only if an antibody to SEQ ID NO1 is detected.

Antibodies to SEQ ID NO1 may be detectable before antibodies to SEQ ID NO2 are. In a preferred embodiment, the presence of antibodies to SEQ ID NO2 is monitored for at least 1, 2, 3 or 4 weeks after a negative result or determined again at least one more later time point to increase the reliability of the result. It has to be borne in mind that IgA and IgM class antibodies emerge before IgG class antibodies do.

In a preferred embodiment, a vaccine has been administrated to the subject, preferably at least 1, 2, 3, 4, 5, 6, 7, 10, 14 days, 3, 4, 6, 8 or 10 weeks prior to the detection of antibodies according to the present invention. It should be mentioned that the precise time when an IgA class antibody to SEQ ID NO1 can be detected first depends on a range of parameters, for example the nature and quantity of the vaccine administered, the immune system of the patient etc. However, an IgA class antibody can generally be detected before an IgG class antibody to SEQ ID NO1.

Preferably the efficacy of a vaccine to be developed or a finalized vaccine such as a commercially available vaccine is assessed by the level of antibody or antibodies detected. A neutralization test may be carried out in addition to determine whether the antibodies produced are neutralizing antibodies (for example the Neutralisa assay by EUROIMMUN Medizinische Labordiagnostika AG, EI 2606-9601-4). If a vaccine or candidate vaccine comprising SEQ ID NO1 or a variant thereof has been administrated and antibodies to SEQ ID NO1 are detectable, but not to SEQ ID NO2, then the vaccine or vaccine candidate is capable of immunizing the subject, more preferably trigger an immune response with antibodies reactive to SARS-CoV-2. If antibodies to SEQ ID NO1 are not detectable after a period of time sufficient to allow for the production of such antibodies, then the vaccine or candidate vaccine is ineffective.

In a preferred embodiment, the vaccine is a composition comprising SEQ ID NO1 or a variant thereof which composition triggers the production of antibodies in the subject, preferably to SEQ ID NO1, only, *i.e.* not to other SARS-CoV-2 proteins, in particular SEQ ID NO2 or SEQ ID NO3 or variants thereof, preferably immunogenic variants. The composition may comprise an adjuvans and a physiologically compatible buffer such as PBS. In a preferred embodiment, the vaccine does not comprise SEQ ID NO2 or SEQ ID NO3 or a variant thereof at a level that would be able to trigger the production of antibodies to SEQ ID NO2. The generation of such pharmaceutical compositions and adjuvants and delivery systems are described in Vaccinology Principles and Practice by Morrow/Sheikh/Schmidt/Davies, Wiley. A range of vaccines are commercially available, for example from Moderna (Jackson et al. An mRNA Vaccine against SARS-CoV-2 - Preliminary Report, N Engl J Med 2020; 383:1920-1931), BionTech/Pfizer (Polack et al. Safety and Efficacy of the BNT162b2 mRNA Covid-19 Vaccine. N Engl J Med. 2020 Dec 31;383(27):2603-2615), AstraZeneca (Watanabe et al. Native-like SARS-CoV-2 spike glycoprotein expressed by ChAdOx1 nCoV-19/AZD1222 vaccine. bioRxiv [Preprint]. 2021 Jan 19:2021.01.15.426463. doi: 10.1101/2021.01.15.426463) and others. A protocol for a vaccination is also disclosed in the examples.

Preferably two or more or three or more shots of vaccine are administered to the subject. A vaccination may be recognized preferably only after two or three or more weeks, after a detectable level of antibodies, initially IgA or IgM class antibodies, followed by IgG class antibodies, is produced by the subject.

In a preferred embodiment, the method or use according to the present invention comprises the step providing a polypeptide comprising SEQ ID NO 1 and a variant thereof and optionally a polypeptide comprising SEQ ID NO2, preferably SEQ ID NO3 and a variant thereof, both polypeptides preferably coated on a diagnostically useful carrier or separate diagnostically useful carriers, and a sample from a subject. The carrier or each carrier may then be contacted with the sample under conditions allowing for binding of any antibodies to SEQ ID NO1, SEQ ID NO2 or SEQ ID NO3. The sample may then be removed and the carrier with may be washed to remove any remaining sample. A secondary antibody or similar reagent or means binding to the antibody and carrying a detectable label may then be contacted with the carrier under conditions allowing formation of a complex between any bound antibody and the secondary antibody. The carrier may be washed then to remove non-bound secondary antibody. Finally, the presence or absence of the antibody is detected by checking whether the secondary antibody may be detected.

In a preferred embodiment, the secondary antibody is an immunoglobulin (Ig), preferably IgG raised in a non-human species, wherein said secondary antibody specifically binds immunoglobulins of one or more specific Ig classes or fragments thereof (e.g. a constant domain of a particular Ig class) of a selected species, preferably human. An example is a polyclonal antibody raised in goat that specifically recognizes human IgA (i.e., a polyclonal goat anti-human IgA antibody). In another preferred embodiment, the secondary antibody is a monoclonal antibody that specifically binds immunoglobulins of one or more specific Ig classes or fragments thereof (e.g. a constant domain of a particular Ig class) of a species, preferably human IgA. Means and methods for producing (mono- or polyclonal) antibodies capable of specific binding of one or more selected target antigens are well known in the art and many are commercially available.

In certain embodiments, the secondary antibody may be chosen to specifically bind to only one, only two, or all three classes of Ig antibodies, i.e. IgA and IgG; or IgA and IgG. In certain embodiments, instead of using only one single kind of secondary antibody, a mixture of several different secondary antibodies may be used, wherein the different secondary antibodies either bind to the same one or different Ig classes (e.g., a mixture of different antibodies (e.g., polyclonal antibodies) all binding to IgA), or to, e.g. IgA and IgG; or IgA and IgM or wherein the different secondary antibodies bind to different individual target structures (e.g. one kind of secondary antibody specifically binding to IgA, and another specifically binding to IgG).

In accordance with the present invention, the term "secondary antibody" in its broadest sense is to be understood to refer to any kind of "binding moiety", preferably binding protein, capable of specific binding to an IgA, IgG and/or IgM class antibody or a fragment thereof such as a constant domain of a particular Ig class of a selected species, preferably human. Non-limiting examples of binding moieties include antibodies, for example antibodies immunologically or genetically derived from any species, for example human, chicken, camel, llama, lamprey, shark, goat, rodent, cow, dog, rabbit, etc., antibody fragments, domains or parts thereof, for example Fab, Fab', F(ab')₂, scFab, Fv, scFv, VH, VHH, VL, VLRs, and the like, diabodies, monoclonal antibodies (mAbs), polyclonal antibodies (pAbs), mAbdAbs, phage display-derived binders, affibodies, heteroconjugate antibodies, bispecific antibodies, evibodies, lipocalins, an-ticalins, affibodies, avimers, maxibodies, heat shock proteins such as GroEL and GroES, trans-bodies, DARPins, aptamers, C-type lectin domains such as tetranectins; human γ-crisstallin and human ubiquitin-derived binders such as affilins, PDZ domain-derived binders; scorpion toxin and/or Kunitz-type domain binders, fibronectin-derived binders such as adnectins, receptors, ligands, lectins, streptavidin, biotin, including derivatives and/or combinations thereof such as bi-/multi-specific formats formed from two or more of these binding molecules. Various antibody-derived and alternative (*i*.*e*. non-antibody) binding protein scaffolds including methods of generation thereof are known in the art (e.g. reviewed in Chiu ML et al., Antibodies (Basel), (2019);8(4):55; Simeon R. & Chen Z., Protein Cell. (2018);9(1):3-14; and Chapter 7 - Non-Antibody Scaffolds from Handbook of Therapeutic Antibodies (2007) edited by Stefan Dübel. Direct reference is made to the description of EP21158065.9, wherein these molecules are defined and their use and generation outlined.

In a preferred embodiment, the antibody to SEQ ID NO: 1 is detected using a labeled secondary antibody, preferably a labeled secondary antibody binding to IgA class antibodies.

As used herein, the term "labeled", with regard to the secondary antibody, is intended to embrace such embodiments wherein the secondary antibody is labelled by coupling, preferably physically linking, a detectable substance, such as a radioactive agent or another molecule providing a detectable signal, such as, without intended to being limiting, a fluorophore, such as a small organic chemical fluorophore such as FITC or fluorescent protein such as GFP, or an enzymatically active label, i.e. an enzyme, such as alkaline phosphatase, whose presence can be assessed and optionally be quantified based on its reactivity with, and/or conversion of, a substrate substance. Various suitable detectable labels are known in the art and some of which are also described herein below.

In a preferred embodiment, the antibody is detected using a method selected from the group comprising colorimetry, immunofluorescence, detection of enzymatic activity, chemiluminscence and radioactivity.

In a preferred embodiment, the diagnostically useful carrier is a line blot (Raoult, D., and Dasch, G. A. (1989), The line blot: an immunoassay for monoclonal and other antibodies. Its application to the serotyping of gram-negative bacteria. J. Immunol. Methods, 125 (1-2), 57-65; WO2013041540).

In another preferred embodiment, the diagnostically useful carrier is a bead. Various beads for numerous applications are commercially available, mainly based on carbohydrate, for example sepharose or agarose, or plastic. They contain active or activatable chemical groups such as a carboxyl or ester group, which can be utilized for the immobilization of a means for specifically capturing an antibody. Preferably, the beads are beads having an average diameter of from 0.1 µm to 10 µm, from 0.5 µm to 8 µm, from 0.75 µm to 7 µm or from 1 µm to 6 µm. The beads can be coated with the means for specifically capturing an antibody directly or *via* affinity ligands, for example biotin or glutathione and streptavidin and GST, respectively. For example, the bead may be coated with biotin or glutathione and the antigen may be fused with streptavidin or glutathione-S-transferase or a variant thereof, respectively. In a preferred embodiment, the bead is coated with a first detectable marker, for example a fluorescent dye.

In a particularly preferred embodiment, the beads are paramagnetic beads, which can be easily concentrated on a surface with the aid of a magnet. A bead may be labeled with a detectable label.

In another preferred embodiment, the carrier is a microtiter plate, preferably comprising at least eight wells that may be used for ELISA. Two or more microtiter plates may be used. At least one of the wells is coated with the polypeptide comprising SEQ ID NO1 or a variant thereof, and optionally at least one other well is coated with the polypeptide comprising SEQ ID NO2 or a variant thereof. At least 3, preferably 4, more preferably 5 calibrators are provided that comprise an antibody to an antigen, preferably both antigens from the group comprising SEQ ID NO1 and SEQ ID NO2 or a variant thereof, at defined concentrations and may be used to set up a calibration curve for semi-quantitative analysis. When the inventive method is carried out, the calibrators may be processed and developed in parallel to the samples. A secondary antibody comprising a detectable label such as an enzymatically active label may be provided, for example a label having horse radish peroxidase activity or alkaline phosphatase activity or an enzyme capable of chemiluminescence.

In another preferred embodiment, the carrier is a microarray. In a preferred embodiment, the term "microarray", as used herein, refers to a chip spotted with a variety of spatially separate antigens, preferably at least 20, preferably 30, 40, 50, 80 or 100. They include SEQ ID NO1 and optionally SEQ ID NO2 or a variant thereof.

The polypeptide, preferably comprising SEQ ID NO1, may be provided in any form and at any degree of purification, from tissues, fluids or cells comprising said polypeptide in an endogenous form, more preferably cells overexpressing the polypeptide, crude or enriched lysates of such cells, to purified and/or isolated polypeptide which may be essentially pure. In a preferred embodiment, the polypeptide is a native polypeptide, wherein the term "native polypeptide", as used herein, refers to a folded polypeptide, more preferably to a folded polypeptide purified from cells, more preferably from prokaryotic or eukaryotic, preferably mammalian cells. A glycosylated form of the polypeptide may be used.

According to the present invention, the polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production using a biotechnological or synthetic approach and is preferably pure, *i.e.* at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

The inventive teachings provide a kit. Such a kit is a container that comprises specific reagents required to practice the inventive method, in particular the diagnostically useful carrier according to the present invention, optionally in addition to one or more solutions or reagents required to practice the inventive method, preferably selected from or all from the group comprising sample dilution buffer, washing buffer and buffer comprising a means or reagent for detecting any specifically captured antibody, such as a secondary antibody which may comprise a detectable label and optionally a means for detecting the latter. Furthermore, it may comprise instructions detailing how to use the kit. It may comprise the diagnostically useful carrier for contacting the inventive polypeptide with a sample from a subject, preferably selected from the group comprising a microtiter plate, two or more beads and a line blot. Furthermore, the kit may comprise one or two positive controls, for example a recombinant or polyclonal antibody known to bind to SEQ ID NO1 and/or another antibody known to bind to SEQ ID NO2, preferably SEQ ID NO3, and a negative control, for example a protein having no detectable affinity to SEQ ID NO1 or SEQ ID NO2 such as bovine serum albumin. The positive control may be a diluted sample from a patient who had been exposed to SARS-CoV-2. A negative control may be a diluted sample from a healthy blood donor without antibodies to SARS-CoV-2. Another positive sample representing a successfully vaccinated subject may be a sample from such a subject or a solution comprising an antibody to SEQ ID NO1 or SEQ ID NO2, but not both, preferably to SEQ ID NO1 only. Finally, such a kit may comprise one or more standard solutions, also referred to as calibrators, comprising one or more antibodies binding to SEQ ID NO1, and may comprise one or more standard solutions, also referred to as calibrator, comprising one or more antibodies binding to SEQ ID NO2, wherein the absolute or relative concentration of the antibody in each standard solution is preferably known. The concentration in the standard solutions may differ such that a concentration range is covered for a calibration curve. For example, the relative concentration of the respective antibody in two standard solutions may be 1:2, 1:5, 1:10, 1:20, 1:50 or 1:100 or more. In a more preferred embodiment, the antibody has a constant region or variant thereof or binding affinity to secondary antibodies like the antibody to be detected and/or any secondary antibody used for the detection binds to said antibody and the antibody to be detected. The antibody may be a monoclonal antibody, preferably a hybrid comprising a human IgA, IgM or IgG or a variant thereof binding to secondary antibodies binding to human IgA, IgM or IgG. The kit may comprise a polypeptide comprising SEQ ID NO1 or a variant thereof, which polypeptide may optionally comprise a detectable label. The kit may comprise a polypeptide comprising SEQ ID NO2 or a variant thereof, which polypeptide may optionally comprise a detectable label. The kit may also comprise a washing solution. The kit may comprise a suitable water-tight vessel for contacting the diagnostically useful carrier with the sample in the presence of other liquids such as a reaction buffer. For example, a line blot may be provided in or in combination with an incubation tray, or a microtiter plate may be provided. Suitable vessels are described in the state of the art, for example EP3025780 or EP3025779. The kit may contain reagents for implementing a method according to the present invention. Calibrators and their use, assays and reagents are described in in The Immunoassay Handbook, 3rd edition, by David Wild, Elsevier 2005, in particular Chapter 9. Such a kit may be taken for the uses according to the present invention.

The antibody to be detected binds preferably specifically to SEQ ID NO1 or SEQ ID NO2, preferably SEQ ID NO3. Specific binding preferably means that the binding reaction is stronger than a binding reaction characterized by a dissociation constant of 1 × 10⁻⁵ M, more preferably 1 × 10⁻⁷ M, more preferably 1 × 10⁻⁸ M, more preferably 1 × 10⁻⁹ M, more preferably 1 × 10⁻¹⁰ M, more preferably 1 × 10⁻¹¹ M, more preferably 1 × 10⁻¹² M, as determined by surface plasmon resonance using Biacore equipment at 25 °C in PBS buffer at pH 7 or ELISA as described in the examples, preferably ELISA.

According to the present invention, a diagnostic device such as the diagnostically useful carrier may be prepared by expressing a recombinant variant of SEQ ID NO1 or SEQ ID NO2, preferably SEQ ID NO3 comprising an affinity tag, optionally with an artificial linker which may include a protease cleavage site, in a cell such as a eukaryotic or prokaryotic cell, contacting the expressed variant with a ligand binding specifically to the affinity tag, which ligand is immobilized on a solid phase, washing the solid phase such that non-specifically bound material from the cell is removed and eluting the expressed variant from the solid phase, preferably by adding an excess of non-immobilized ligand. The variant may then be immobilized on the device. Optionally, the affinity tag may be removed by contacting the variant with a protease, preferably a protease recognizing the protease cleavage site, before the immobilization. The affinity tag may be selected from the group of tags comprising 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV and Xpress Tag. Useful proteases include, but are not limited to TEV, Thrombin, Faktor Xa or Enteropeptidase. Suitable linkers are part of vectors, for example pET vector series (Novagen).

The methods according to the present invention may also be used for screening whether donated blood comprises antibodies to SARS-CoV-2 and may be used to prepare a medicament used to treat a patient suffering from SARS-CoV-2 and who may benefit from the administration of such antibodies. In particular, blood comprising an antibody to SEQ ID NO1 and an antibody to SEQ ID NO2 is from a patient having been exposed to SARS-CoV-2. This may have been the results of an active infection or a vaccination using an inactivated virus or SEQ ID NO1 and SEQ ID NO2 or a variant thereof.

The teachings of the present invention may not only be carried out using polypeptides having the exact sequences referred to in this application explicitly, for example by function, name, sequence or accession number, or implicitly, but also using variants of such polypeptides.

In a preferred embodiment, the term "variant", as used herein, may refer to at least one fragment of the full length sequence referred to or a polypeptide comprising said fragment, more specifically to one or more amino acid or nucleic acid sequences which are, relative to the full-length sequence, truncated at one or both termini by one or more amino acids. Such a fragment comprises or encodes for a peptide having at least 10, 15, 25, 50, 75, 100, 150, 200, 250, 300, 400, 500 or 600 successive amino acids of the original sequence or for a variant thereof.

In another preferred embodiment, the term "variant" relates not only to at least one fragment, but also a polypeptide or a fragment thereof comprising amino acid sequences, preferably a fragment comprising at least 25, more preferably 50, more preferably 200 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind specifically to an antibody of interest, or the fold or structure of the polypeptide are deleted or substituted and/or one or more such essential amino acids are replaced in a conservative manner and/or amino acids are added or deleted such that the biological activity of the polypeptide is at least partially preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal Wand Clustal X version 2.0. Bioinformatics, 23, 2947-2948) is used applying default settings.

SARS-CoV-2-related publications on specific amino acid sequences such as Beal et al. may aid the skilled one in designing variants (Beal, J., Mitechell, T., Wyschogrod, W., Manthey, J, and Clore, A. (2020) Highly Distinguished Amino Acid Sequences of 2019-nCoV (Wuhan Coronavirus) doi: https://doi.org/10.1101/2020.01.31.929497, as well as publications relating to SARS-CoV, for example Hua, R., Zhou, Y., Wang, Y., Hua, Y and Tong, T. (2004) Identification of two antigenic epitopes on SARS-CoV spike protein, BBR 319, 929-935, wherein homologous epitopes may be found and SARS-CoV-2 epitopes be identified on account of their homology. Dahlke, C., Heidepriem, J., Kobbe, R., Santer, R., Koch, T., Fathi, A., Ly, M. L., Schmiedel, S., Seeberger, P. H., and Loeffler, F. (2020), medRxiv, https://doi.org/10.1101/2020.04.14.20059733 contacted a peptide microarray with patient samples and identified reactive epitopes.

In a preferred embodiment, variants may, in addition, comprise chemical modifications, for example labels such as isotopic labels or detectable labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof, for example artificial linkers, affinity tags, other antigens and the like. For example, SEQ ID NO4 is a fusion protein according to the present invention.

The variant of the polypeptide has biological activity. In a preferred embodiment such biological activity is the ability to bind to the respective antibody such as an antibody to SEQ ID NO1 or an antibody to SEQ ID NO2, preferably SEQ ID NO3. In a preferred embodiment it comprises an epitope having the ability or has itself the ability to bind to the antibody to be detected, preferably from a sample from a patient suffering from SARS-CoV-2 or a patient vaccinated with a vaccine comprising SEQ ID NO1, wherein more preferably the epitope comprises a sequence comprising a stretch of at least 5, 6, 7 or 8 amino acid residues from the wildtype sequence, more specifically SEQ ID NO1 or an antibody to SEQ ID NO2. More preferably, it does not bind specifically to homologues from other coronaviruses, preferably coronaviruses other than SARS-CoV, more preferably from the group comprising NL63, 229E, OC43, HKU1.

The detection of the antibody or complex for the methods according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques. In a preferred embodiment, the complex, preferably comprising an antibody to SEQ ID NO1, more preferably IgA class, and a polypeptide comprising SEQ ID NO1 or a variant thereof, is detected using a method selected from the group comprising immunodiffusion techniques, immunoelectrophoretic techniques, light scattering immunoassays, agglutination techniques, labeled immunoassays from the group comprising radiolabeled immunoassays, chemiluminscence immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14. Preferably the test format is an ELISA and a microtiter plate comprising wells is used as a diagnostically useful carrier. In a preferred embodiment, the term "presence" of an antibody, as used herein, means that the antibody is detectable using such a method, preferably a method selected from the group comprising enzyme immunoassays, more preferably colorimetric assays or chemiluminescence immunoassays, most preferably colorimetric assays based on line blot and detection using alkaline phosphatase activity as described in the examples, even more preferably using the ELISA Kit (EI 2606-9601 A) by EUROIMMUN Medizinische Labordiagnostika AG.

Preferably the first and, if present, the second polypeptide or a variant thereof is immobilized on a solid phase of the carrier. It may be directly immobilized on the solid phase when contacted with the sample, but a competitive assay, a capture bridge assay, an immunometric assay, a class-specific second antibody on the solid phase, a class capture assay, direct or indirect may also be used. The principle of each of these formats is detailed in The Immunoassay Handbook, 3rd edition, edited by David Wild, Elsevier, 2005. More preferably, the solid phase is a test strip or a well of a microtiter plate for ELISA or a bead, preferably a well of a microtiter plate for ELISA. The first and the second polypeptide may be on the same or on different carriers.

In a preferred embodiment, a competitive assay format is used, wherein the antibody to be detected competes with another antibody to SEQ ID NO: 1 or another ligand binding specifically to SEQ ID NO: 1. The ligand binding specifically to SEQ ID NO: 1 may be selected from the group comprising an aptamer or antibody binding to SEQ ID NO: 1 and the human ACE2 receptor (SEQ ID NO: 39) or a variant thereof, the natural binding partner of the SARS-CoV-2 spike protein. Such a method may comprise providing a polypeptide comprising SEQ ID NO: 1 or variant thereof and the ligand binding specifically to SEQ ID NO: 1, preferably from the group comprising an antibody, an aptamer and the ACE receptor or a variant thereof. If the antibody to be detected is present, it will interfere with the formation of the complex or partially or fully displace the ligand binding specifically to SEQ ID NO: 1, thus reducing the number of the complexes. Any complex comprising the antibody to be detected may then be detected by precipitating the complex, for example using an affinity ligand attached to the polypeptide comprising SEQ ID NO: 1 or to a molecule binding to the antibody to be detected such as a secondary antibody. Examples of affinity ligands include glutathione and biotin. A binding partner of the affinity ligand may be coated on a solid phase which binds to the affinity ligand such as GST or streptavidin, respectively. Any complex precipitated may then be detected, preferably by detecting a detectable label attached to the ligand binding specifically to the polypeptide comprising SEQ ID NO: 1 or attached to the polypeptide comprising SEQ ID NO: 1, respectively. A specific competitive test has been published by Tan et al. (2020) A SARS-CoV-2 surrogate virus neutralization test based on antibody-mediated blockage of ACE2-spike protein protein interaction, Nature Biotechnology 38 (1073-1078).

Alternatively, the complex may be formed on the surface of a carrier if the ligand binding specifically to SEQ ID NO: 1 or the polypeptide comprising SEQ ID NO: 1 or a variant is coated on said surface. Specific IgA class antibodies may be detected by isolating the antibodies of the Ig class of interest before, preferably IgAfor example using a secondary antibody.

In another preferred embodiment, a complex comprising a first and a second polypeptide comprising SEQ ID NO: 1 or a variant thereof, such as the receptor binding domain (RBD), and the antibody to be detected may be formed in a liquid phase if the antibody is present in the sample, since the antibody has two or more binding sites, each binding to a polypeptide comprising SEQ ID NO: 1. Any complex comprising the antibody to be detected may then be detected by immobilizing, preferably precipitating the complex, for example using an affinity ligand attached to the first polypeptide such as glutathione or biotin and a binding partner coated on a solid phase which binds to the affinity ligand such as GST or streptavidin, respectively, which solid phase may for example be a bead. Any complex immobilized or precipitated may then be detected, preferably by detecting a detectable label attached to the second polypeptide. Alternatively, the complex may be formed on the surface of a carrier if the first polypeptide comprising SEQ ID NO: 1 or a variant thereof is immobilized on said surface. Alternatively, the first and the second polypeptide may each be labeled with two different labels which are detectable only if they are in close proximity, for example when bridged by the antibody to be detected. Again, the presence or absence of IgA, IgM and IgG class antibodies will be detected unless the specific Ig class antibodies have been isolated before, preferably from the group comprising IgA, IgM and IgG class antibodies, preferably IgA, for example using Protein A or Protein G or a secondary antibody. Preferably, in an early phase of the infection, when IgG antibodies have not yet been produced, IgA antibodies will predominantly or only be detected.

In a preferred embodiment, the first and the second polypeptide are spatially separate such that a signal indicating the presence of an antibody to SEQ ID NO1 and a signal indicating the presence of an antibody to SEQ ID NO2, preferably SEQ ID NO3 can be distinguished. For example, the first and the second polypeptides may be on different beads, on different wells of one or more microtiter plates or spatially separate spots on a membrane such as a blot membrane, for example Western blot, dot blot or line blot. The membrane may optionally be on a microtiter plate well.

In a preferred embodiment, a vaccine comprising SEQ ID NO1 or a variant thereof had been administered to a subject, preferably by injection.

The present invention comprises a range of polypeptide sequences, more specifically
SEQ ID NO1 (SARS-CoV-2 S1 Spike-Protein) 
VNLTTRTQLPPAYTNSFTRGVYYPDKVFRSSVLHSTQDLFLPFFSNVTWFHAIHVSGTNGTK
RFDNPVLPFNDGVYFASTEKSNIIRGWIFGTTLDSKTQSLLIVNNATNVVIKVCEFQFCNDPFL
GVYYHKNNKSWMESEFRVYSSANNCTFEYVSQPFLMDLEGKQGNFKNLREFVFKNIDGYF
KIYSKHTPINLVRDLPQGFSALEPLVDLPIGINITRFQTLLALHRSYLTPGDSSSGWTAGAAAY
YVGYLQPRTFLLKYNENGTITDAVDCALDPLSETKCTLKSFTVEKGIYQTSNFRVQPTESIVR
FPNITNLCPFGEVFNATRFASVYAWNRKRISNCVADYSVLYNSASFSTFKCYGVSPTKLNDL
CFTNVYADSFVIRGDEVRQIAPGQTGKIADYNYKLPDDFTGCVIAWNSNNLDSKVGGNYNYL
YRLFRKSNLKPFERDISTEIYQAGSTPCNGVEGFNCYFPLQSYGFQPTNGVGYQPYRVVVL
SFELLHAPATVCGPKKSTNLVKNKCVNFNFNGLTGTGVLTESNKKFLPFQQFGRDIADTTDA
VRDPQTLEILDITPCSFGGVSVITPGTNTSNQVAVLYQDVNCTEVPVAIHADQLTPTWRVYST
GSNVFQTRAGCLIGAEHVNNSYECDIPIGAGICASYQTQTNSPRRAR
SEQ **DI NO2** SARS-CoV-2 nucleoprotein (full length) (data base code: YP_009724397.2) 
MSDNGPQNQRNAPRITFGGPSDSTGSNQNGERSGARSKQRRPQGLPNNTASWFTALTQH
GKEDLKFPRGQGVPINTNSSPDDQIGYYRRATRRIRGGDGKMKDLSPRWYFYYLGTGPEA
GLPYGANKDGIIWVATEGALNTPKDHIGTRNPANNAAIVLQLPQGTTLPKGFYAEGSRGGSQ
ASSRSSSRSRNSSRNSTPGSSRGTSPARMAGNGGDAALALLLLDRLNQLESKMSGKGQQ
QQGQTVTKKSAAEASKKPRQKRTATKAYNVTQAFGRRGPEQTQGNFGDQELIRQGTDYKH
WPQIAQFAPSASAFFGMSRIGMEVTPSGTWLTYTGAIKLDDKDPNFKDQVILLNKHIDAYKTF
PPTEPKKDKKKKADETQALPQRQKKQQTVTLLPAADLDDFSKQLQQSMSSADSTQA
SEQ ID NO3 SARS-CoV-2 nucleoprotein (118-419), basis for designing the polypeptide used in Example 1 
GLPYGANKDGIIWVATEGALNTPKDHIGTRNPANNAAIVLQLPQGTTLPKGFYAEGSRGGSQ
ASSRSSSRSRNSSRNSTPGSSRGTSPARMAGNGGDAALALLLLDRLNQLESKMSGKGQQ
QQGQTVTKKSAAEASKKPRQKRTATKAYNVTQAFGRRGPEQTQGNFGDQELIRQGTDYKH
WPQIAQFAPSASAFFGMSRIGMEVTPSGTWLTYTGAIKLDDKDPNFKDQVILLNKHIDAYKTF
PPTEPKKDKKKKADETQALPQRQKKQQTVTLLPAADLDDFSKQLQQSMSSADSTQA
SEQ **ID** NO4 (SARS-CoV-2 Spike-Protein, C-terminally his-tagged, as after cleavage of signal peptide, used in the examples)

VNLTTRTQLPPAYTNSFTRGVYYPDKVFRSSVLHSTQDLFLPFFSNVTWFHAIHVSGTNGTK
RFDNPVLPFNDGVYFASTEKSNIIRGWIFGTTLDSKTQSLLIVNNATNVVIKVCEFQFCNDPFL
GVYYHKNNKSWMESEFRVYSSANNCTFEYVSQPFLMDLEGKQGNFKNLREFVFKNIDGYF
KIYSKHTPINLVRDLPQGFSALEPLVDLPIGINITRFQTLLALHRSYLTPGDSSSGWTAGAAAY
YVGYLQPRTFLLKYNENGTITDAVDCALDPLSETKCTLKSFTVEKGIYQTSNFRVQPTESIVR
FPNITNLCPFGEVFNATRFASVYAWNRKRISNCVADYSVLYNSASFSTFKCYGVSPTKLNDL
CFTNVYADSFVIRGDEVRQIAPGQTGKIADYNYKLPDDFTGCVIAWNSNNLDSKVGGNYNYL
YRLFRKSNLKPFERDISTEIYQAGSTPCNGVEGFNCYFPLQSYGFQPTNGVGYQPYRVVVL
SFELLHAPATVCGPKKSTNLVKNKCVNFNFNGLTGTGVLTESNKKFLPFQQFGRDIADTTDA
VRDPQTLEILDITPCSFGGVSVITPGTNTSNQVAVLYQDVNCTEVPVAIHADQLTPTWRVYST
GSNVFQTRAGCLIGAEHVNNSYECDIPIGAGICASYQTQTNSPRRARLEHHHHHHHH

### Example 1: Preparation of antigen

To prepare SEQ **ID** NO4, cell culture supernatant was adjusted to 5 mmol/l tris chloride pH 8.0, 164 mmol/l sodium chloride, 50 mmol/l magnesium chloride, 20 mmol/l imidazole, 0,1% Triton X-100, cleared by centrifugation for 30 minutes at 17,600xg, 4°C, applied to Nickel Rapid Run (Agarose Bead Technologies, Miami, FL, USA) equilibrated with 5 mmol/l tris chloride pH 8.0, 300 mmol/l sodium chloride, 20 mmol/l imidazole and eluted by increasing the imidazole concentration to 150 mmol/l. All fractions containing SEQ ID NO3 were pooled and concentrated by ultrafiltration (VivaSpin, Sartorius, Göttingen, Germany). The final preparation was stored at -80°C until further use.

The final protein preparation of SEQ ID NO4 was treated with or without 16 mmol/l dithiotreitol and incubated at 70°C or at room temperature for 10 minutes, followed by SDS gel electrophoresis and Coomassie staining.

Protein identity was verified by mass spectrometry.

### Example 2: Vaccination studies

A healthy subject received on day 1 an injection into the musculus quadriceps of 12.86 µg recombinant S1 protein in physiological PBS (SEQ ID NO4). Alum adjuvans (Twinrix for adults, EMRA-MED Arzneimittel GmbH) was applied according to the manufacturer's instructions. On days 9, 21 and 28, the subject received an injection of another 12.86 µg S1 protein in physiological PBS buffer and 10 µl Imject alaun adjuvans in 500 µl sodium chlorid solution.

Blood samples were obtained on days 10, 23 and 29.

The presence of IgG and IgA antibodies to SARS-CoV-2 S1 and nucleoprotein was determined in serum samples from the healthy subject using serological kits (EUROIMMUN Medizinische Labordiagnostika AG, EI 2606-9601 A, EI 2606-9601 G, EI 2606-9601-2 G and EI 2606-9601-2 M) according to the manufacturer's instructions. Briefly, the assays are based on the capture of a human antibody to an antigen such as SEQ ID NO1 using a polypeptide comprising the antigen coated on an ELISA microtiter plate. The technology is described in more detail in EP3715847 A1. Briefly, secondary antibody recognizing the class of the antibody to be detected (for example IgA) with an enzymatically active label is used to detect the antibody of interest. It converts a chromogenic substrate until the reaction is stopped by addition of a stop solution, followed by measuring the absorption of the solution in each ELISA well.

As controls, samples from healthy blood donors and from patients suffering from SARS-CoV-2 infection were used.

**Table 1: Antibodies to SARS-CoV-2 antigens in a subject vaccinated using S1 protein**

| Day | IgA (S1) (Cut off: <0.8) | IgG (S1) (Cut off: <0.8) | IgG (NCP) (Cut off: <0.8) | IgM (NCP) (Cut off: <0.8) |
|---|---|---|---|---|
| 10 | 0.5 | 0.5 | - | - |
| 23 | **1.0 (!)** | 0.2 | - | - |
| 29 | **3.5** | **6.2** | 0.6 | 0.7 |

In two patients suffering from SARS-CoV-2 infection (positive controls), samples obtained 17 and 19 days after the onset of symptoms (usually 5-6 days after the infection). Antibody titers are shown in Table 2.

**Table 2: Antibodies to SARS-CoV-2 antigens in two patients suffering from SARS-CoV-2 infection**

| Day | IgA (S1) (Cut off: <0.8) | IgG (S1) (Cut off: <0.8) | IgG (NCP) (Cut off: <0.8) | IgM (NCP) (Cut off: <0.8) |
|---|---|---|---|---|
| Patient 1 | 2,6 | 1,8 | 1,7 | 1,3 |
| 1,3 | 1.3 | 6.3 | 3.5 | 11.4 |

In four healthy subjects who did not receive any vaccination (negative controls), samples were obtained. Antibody titers are shown in Table 3.

**Table 3: Antibodies to SARS-CoV-2 antigens in healthy subjects**

| Day | IgA (S1) (Cut off: <0.8) | IgG (S1) (Cut off: <0.8) | IgG (NCP) (Cut off: <0.8) | IgM (NCP) (Cut off: <0.8) |
|---|---|---|---|---|
| Patient 1 | 0,0 | 0,1 | 0,0 | 0,5 |
| Patient 2 | 0,2 | 0,3 | 0,1 | 0,4 |
| Patient 3 | 0,1 | 0,1 | 0,1 | 0,2 |
| Patient 4 | 0,1 | 0,1 | 0,1 | 0,1 |

These results show that vaccinated and infected subjects can be distinguished. Antibodies to the S1 antigen comprised in the vaccine can be detected in both, but antibodies to the nucleocapsid protein only in infected patients, but not vaccinated subjects.

### Example 2: Detection of IgA in a larger cohort of patients after vaccination

In a cohort comprising 64 patients vaccinated as described in Example 1, the presence or absence of IgA antibodies to SEQ ID NO1 and IgG/IgM class antibodies to SARS-CoV-2 nucleocapsid protein was detected using the methods described in Example 2.

An IgA response to SEQ ID NO1 could be detected in 54/64 individuals. An IgG or IgM response to nucleocapsid protein could each be detected only in one person; in both cases the person had no IgA response.

It should be mentioned that some patients do not develop any antibodies as a result of a vaccination or even genuine infection. However, the fact that approximately 85% of the patients were IgA positive shows a very good sensitivity of the assay. Using saliva samples rather than blood samples in select samples, the inventors could detect a very high IgA response.

The specificity of the assay is also very high. According to the manual of EI 2606-9601 A, the specificity is more than 98 %, based on tests with samples from panels including a range of subject groups, including patients infected with seasonal corona viruses and more than 800 blood donors.

### Example 3: Kinetics of IgA/IgG antibody response following vaccination

In a cohort of 16 patients vaccinated with the Moderna vaccine (COVID-19 Vaccine Moderna ^{®}) in 2021, at least three samples were taken, one 7 day and at least two samples taken 14, 23 and/or 35 days after the administration of the vaccine. The presence or absence of IgA and IgG class antibodies to SEQ ID NO1 was detected using the methods described in Example 1, with the exception that the EUROIMMUN SARS-2-QuantiVac (EI-2606-10 G), which allows the quantification of IgG, was used rather than product El-2606 G. The SARS-CoV-2 NeutraLISA (EI 2606-4) was performed in addition. The manufacturer's instructions were followed regardless of which kit was used.

**Table 1 shows a set of representative data from a patient:**

| Pat.-ID | Days after 1. vaccination | **Results** | | | | |
|---|---|---|---|---|---|---|
| | | **EUROIMMUN ELISA** | | | **EUROIMMUN NeutraLISA** | |
| | | **SARS-CoV-2** | **SARS-2 QuantiVac** | | **SARS-CoV-2** | |
| | | EI 2606 A | EI 2606-10 G | | EI 2606-4 | |
| | | various | various | | C201201DB | |
| | | **IgA** | **IgG** | | sVNT | |
| | | Ratio | RU/ml | BAU/ml | OD | % IH |
| | | **pos: ≥ 1,1** | **pos: ≥ 11** | **pos:** ≥ **35,2** | | **pos: ≥ 35** |
| | | bl: 0,8-<1,1 | bl: 8-<11 | bl: 25,6-<35,2 | | bl: 20-<35 |
| | | | | | | |
| 3 | 7 | **5,0** | 2 | 6 | 1,684 | 8 |
| 3 | 14 | **> 7,0** | **82** | **262** | 0,640 | **65** |
| 3 | 23 | **6,0** | **98** | **314** | 0,607 | **67** |
| 3 | 35 | **> 7,0** | **> 120** | **> 384** | 0,008 | **100** |

In none of the samples could IgG be detected before IgA. In 4/16 samples, IgA, but not IgG could be detected as early as 7 days after the administration of the vaccine. In another sample, IgA and IgG, the latter only just above the cut off value, could be detected. None of the samples that were taken first or in which any antibody could be first detected comprises detectable levels of IgG, but not IgA. Once IgA emerged, it could be also be detected in any samples obtained later from the same subject, confirming that the IgA immune response is persisting, but contrast to IgM, which is detectable only for few days, a short time window often missed by serological testing.

In two patients, samples obtained on day 7 showed values of 5.0 or more, well above the cut off value (0.8 for borderline result, 1.1 for a positive result), strongly suggesting that antibodies could have been detected well before day 7.

These results prove that IgA indicates a successful vaccination earlier than IgG in a significant proportion of samples, consistent with publications by independent scientists. While it may not be possible to demonstrate the vaccination in *all* subjects at this early point, a significant proportion could be shown to have antibodies. Considering the considerable impact of quarantines on the economic situation of companies and personal lives of citizens, being able to detect the vaccination in at least 31.5 % of the subjects is an enormous advantage and may end unnecessary restrictions for many people and save companies considerable resources.

## Claims

1. A method for distinguishing an immunized healthy subject, which is a vaccinated subject, from a non-immunized healthy subject comprising the step determining in a blood sample the presence or absence of an IgA class antibody to SEQ ID NO1, wherein the sample comprises a set of representative antibodies from the subject, and wherein the blood sample is from a human subject.

2. A method for testing the efficacy of a vaccine, comprising the step determining in a blood sample the presence or absence of an IgA class antibody to SEQ ID NO1, wherein the sample comprises a set of representative antibodies from the subject, and wherein the blood sample is from a human subject.

3. A use of a kit for distinguishing an immunized subject, which is a vaccinated subject, from a non-immunized subject, wherein the kit comprises a carrier coated with a polypeptide comprising SEQ ID NO1 or a variant thereof and a means for detecting a specifically captured antibody
wherein
a. the polypeptide comprises the full length-sequence of SEQ ID NO1 or a variant of SEQ ID NO1, which has a sequence identity to SEQ ID NO1 of at least 80, 85, 90, 92, 94,95, 96, 97, 98, 99 %, optionally fused to one or more artificial linkers, affinity tags and/or other antigens, or
b. the variant is a truncated SEQ ID NO1, i. e. less than the full-length sequence of SEQ ID NO1, wherein the truncation is at the N-terminus, C-terminus or both, optionally fused to one or more artificial linkers and/or affinity tags, and
i. comprises a fragment of SEQ ID NO1 comprising at least 25, 50, 75, 100, 150, 200, 250, 300, 400, 500 or 600 successive amino acids of SEQ ID NO1 and/or
ii. comprises a fragment of SEQ ID NO1 comprising at least 200 successive amino acids with a sequence identity to the reference fragment of SEQ ID NO1 of at least 80, 85, 90, 92, 94,95, 96, 97, 98, or 99 %,
and wherein the variant has the ability to bind to an antibody to SEQ ID NO1 from a sample from a patient suffering from a SARS-CoV-2 infection,
comprising the step determining in a blood sample the presence or absence of an IgA class antibody to SEQ ID NO1, wherein the sample comprises a set of representative antibodies from the subject.

4. The method or use according to any of claims 1 or 3, wherein a non-immunized healthy, an immunized healthy and an infected subject is distinguished.

5. The method according to any of claims 1 to 2 or 4,
wherein a carrier coated with a polypeptide comprising SEQ ID NO1 or a variant thereof is contacted with the sample under conditions allowing for binding of any antibodies to SEQ ID NO1, and a secondary antibody or means binding to the IgA class antibody and carrying a detectable label is contacted with the carrier under conditions allowing formation of a complex between any bound antibody and the secondary antibody or means,
wherein
a. the polypeptide comprises the full length-sequence of SEQ ID NO1 or a variant of SEQ ID NO1, which has a sequence identity to SEQ ID NO1 of at least 80, 85, 90, 92, 94,95, 96, 97, 98, 99 %, optionally fused to one or more artificial linkers, affinity tags and/or other antigens, or
b. the variant is a truncated SEQ ID NO1, i. e. less than the full-length sequence of SEQ ID NO1, wherein the truncation is at the N-terminus, C-terminus or both, optionally fused to one or more artificial linkers and/or affinity tags, and
i. comprises a fragment of SEQ ID NO1 comprising at least 25, 50, 75, 100, 150, 200, 250, 300, 400, 500 or 600 successive amino acids of SEQ ID NO1 and/or
ii. comprises a fragment of SEQ ID NO1 comprising at least 200 successive amino acids with a sequence identity to the reference fragment of SEQ ID NO1 of at least 80, 85, 90, 92, 94,95, 96, 97, 98, or 99 %,
and wherein the variant has the ability to bind to an antibody to SEQ ID NO1 from a sample from a patient suffering from a SARS-CoV-2 infection.

6. The method or use according to any of claim 1 to 5, wherein the carrier is selected from the group comprising a bead, a test strip, a microtiter plate, a microarray, a blot, preferably from the group comprising western blot, line blot and dot blot, a glass surface, a slide, a biochip and a membrane, and is most preferably a microtiter plate or a line blot.

7. The method or use according to any of claims 1 to 6, wherein an IgA class antibody to SEQ ID NO1 is detected before the emergence of IgG class antibodies to SEQ ID NO1.

8. The method or use according to any of claims 1 to 7, wherein the presence or absence of an antibody from the group of classes comprising IgG, IgA and IgM to SEQ ID NO1 is detected.

9. The method or use according to any of claims 1 to 8, wherein the presence or absence of an antibody to SEQ ID NO1 is monitored for at least 1, 2, 3 or 4 weeks.

10. The method or use according to any of claims 1 to 9, wherein a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminescence immunoassays and immunofluorescence techniques is used to detect the antibody.

11. The method or use according to any of claims 1 to 10, wherein a method from the group comprising a competitive assay, a capture bridge assay, an immunometric assay, a class-specific second antibody on the solid phase, a class capture assay and an ELISA is used to detect the antibody.

12. The method or use according to any of claims 1 to 11, wherein the sample is a blood sample from the group comprising serum, plasma and whole blood.

13. The method or use according to any of claims 1 to 12, wherein a vaccination may be recognized by the production of a detectable level of antibodies.

14. The method or use according to claim 13, wherein the detectable level of antibodies comprises IgA, IgM and IgG class antibodies to SEQ ID NO1.

15. The method or use according to any of claims 1 to 14, wherein two or three or more shots of vaccine were administered to the subject.

16. The method or use according to any of claims 1 to 15, wherein a means binding to the IgA class antibody is used which is (a) a secondary antibody binding to human IgA class antibodies or (b) a labeled polypeptide which comprises SEQ ID NO1 or a variant thereof which binds to an antibody from the group of classes comprising IgG, IgA and IgM to SEQ ID NO1,
wherein
a. the polypeptide comprises the full length-sequence of SEQ ID NO1 or a variant of SEQ ID NO1, which has a sequence identity to SEQ ID NO1 of at least 80, 85, 90, 92, 94,95, 96, 97, 98, 99 %, optionally fused to one or more artificial linkers, affinity tags and/or other antigens, or
b. the variant is a truncated SEQ ID NO1, i. e. less than the full-length sequence of SEQ ID NO1, wherein the truncation is at the N-terminus, C-terminus or both, optionally fused to one or more artificial linkers and/or affinity tags, and
i. comprises a fragment of SEQ ID NO1 comprising at least 25, 50, 75, 100, 150, 200, 250, 300, 400, 500 or 600 successive amino acids of SEQ ID NO1 and/or
ii. comprises a fragment of SEQ ID NO1 comprising at least 200 successive amino acids with a sequence identity to the reference fragment of SEQ ID NO1 of at least 80, 85, 90, 92, 94,95, 96, 97, 98, or 99 %,
and wherein the variant has the ability to bind to an antibody to SEQ ID NO1 from a sample from a patient suffering from a SARS-CoV-2

## Patentansprüche

1. Verfahren zum Unterscheiden eines immunisierten, gesunden Subjektes, wobei es sich um ein geimpftes Subjekt handelt, von einem nicht immunisierten, gesunden Subjekt, umfassend den Schritt Nachweisen der Anwesenheit oder Abwesenheit eines Antikörpers der Immunglobulinklasse IgA gegen SEQ ID NO1 in einer Blutprobe, wobei die Probe eine Reihe repräsentativer Antikörper aus dem Subjekt umfasst, und wobei die Blutprobe von einem menschlichen Subjekt stammt.

2. Verfahren zum Testen der Wirksamkeit eines Impfstoffs, umfassend den Schritt Nachweisen der Anwesenheit oder Abwesenheit eines Antikörpers der Immunglobulinklasse IgA gegen SEQ ID NO1 in einer Blutprobe, wobei die Probe eine Reihe repräsentativer Antikörper aus dem Subjekt umfasst, und wobei die Blutprobe von einem menschlichen Subjekt stammt.

3. Verwendung eines Kits zum Unterscheiden eines immunisierten Subjektes, das ein geimpftes Subjekt ist, von einem nicht immunisierten Subjekt, wobei das Kit einen Träger, der mit einem Polypeptid beschichtet ist, das SEQ ID NO1 oder eine Variante davon umfasst, und ein Mittel zum Nachweis eines spezifisch eingefangenen Antikörpers umfasst
wobei
a. das Polypeptid die Volllängensequenz von SEQ ID NO1 oder eine Variante von SEQ ID NO1 umfasst, die eine Sequenzidentität zu SEQ ID NO1 von mindestens 80, 85, 90, 92, 94, 95, 96, 97, 98, 99 % aufweist, optional fusioniert mit einem oder mehr als einem künstlichen Linker, einer oder mehr als einer Affinitätsmarkierung und/oder einem oder mehr als einem anderen Antigen, oder
b. die Variante eine verkürzte SEQ ID NO1 ist, d.h. weniger als die Volllängensequenz von SEQ ID NO1, wobei die Verkürzung am N-Terminus, C-Terminus oder an beiden vorliegt, optional fusioniert mit einem oder mehr als einem künstlichen Linker und/oder Affinitätstag, und
i. ein Fragment von SEQ ID NO1 umfasst, das mindestens 25, 50, 75, 100, 150, 200, 250, 300, 400, 500 oder 600 aufeinanderfolgende Aminosäuren von SEQ ID NO1 umfasst und/oder
ii. ein Fragment von SEQ ID NO1 umfasst, das mindestens 200 aufeinanderfolgende Aminosäuren mit einer Sequenzidentität zum Referenzfragment von SEQ ID NO1 von mindestens 80, 85, 90, 92, 94, 95, 96, 97, 98 oder 99 % umfasst,
und wobei die Variante die Fähigkeit hat, an einen Antikörper gegen SEQ ID NO1 aus einer Probe eines Patienten zu binden, der an einer SARS-CoV-2-Infektion leidet,
umfassend den Schritt Nachweisen der Anwesenheit oder Abwesenheit eines Antikörpers der Immunglobulinklasse IgA gegen SEQ ID NO1 in einer Blutprobe, wobei die Probe eine Reihe repräsentativer Antikörper aus dem Subjekt umfasst.

4. Verfahren oder Verwendung nach einem der Ansprüche 1 oder 3, wobei zwischen einem nicht immunisierten gesunden, einem immunisierten gesunden und einem infizierten Subjekt unterschieden wird.

5. Verfahren nach einem der Ansprüche 1 bis 2 oder 4,
wobei ein Träger, der mit einem Polypeptid beschichtet ist, das SEQ ID NO1 oder eine Variante davon umfasst, mit der Probe unter Bedingungen in Kontakt gebracht wird, die eine Bindung von irgendwelchen Antikörpern an SEQ ID NO1 ermöglichen, und ein sekundärer Antikörper oder ein Mittel, das an den Antikörper der Immunglobulinklasse IgA bindet und eine nachweisbare Markierung trägt, mit dem Träger unter Bedingungen in Kontakt gebracht wird, die die Bildung eines Komplexes zwischen irgendeinem gebundenen Antikörper und dem sekundären Antikörper oder Mittel ermöglichen,
wobei
a. das Polypeptid die Volllängensequenz von SEQ ID NO1 oder eine Variante von SEQ ID NO1 umfasst, die eine Sequenzidentität zu SEQ ID NO1 von mindestens 80, 85, 90, 92, 94, 95, 96, 97, 98, 99 % aufweist, optional fusioniert mit einem oder mehr als einem künstlichen Linker, einer oder mehr als einer Affinitätsmarkierung und/oder einem oder mehr als einem anderen Antigen, oder
b. die Variante eine verkürzte SEQ ID NO1 ist, d.h. weniger als die Volllängensequenz von SEQ ID NO1, wobei die Verkürzung am N-Terminus, C-Terminus oder an beiden vorliegt, optional fusioniert mit einem oder mehr als einem künstlichen Linker und/oder Affinitätstag, und
i. ein Fragment von SEQ ID NO1 umfasst, das mindestens 25, 50, 75, 100, 150, 200, 250, 300, 400, 500 oder 600 aufeinanderfolgende Aminosäuren von SEQ ID NO1 umfasst und/oder
ii. ein Fragment von SEQ ID NO1 umfasst, das mindestens 200 aufeinanderfolgende Aminosäuren mit einer Sequenzidentität zum Referenzfragment von SEQ ID NO1 von mindestens 80, 85, 90, 92, 94, 95, 96, 97, 98 oder 99 % umfasst,
und wobei die Variante die Fähigkeit hat, an einen Antikörper gegen SEQ ID NO1 aus einer Probe eines Patienten zu binden, der an einer SARS-CoV-2-Infektion leidet.

6. Verfahren oder Verwendung nach einem der Ansprüche 1 bis 5, wobei der Träger ausgewählt ist aus der Gruppe umfassend ein Bead, einen Teststreifen, eine Mikrotiterplatte, ein Microarray, einen Blot, bevorzugt aus der Gruppe umfassend Western Blot, Line Blot und Dot Blot, eine Glasoberfläche, einen Objektträger, einen Biochip und eine Membran, und am bevorzugtesten eine Mikrotiterplatte oder ein Line Blot ist.

7. Verfahren oder Verwendung nach einem der Ansprüche 1 bis 6, wobei ein Antikörper der Immunglobulinklasse IgA gegen SEQ ID NO1 vor dem Auftreten von Antikörpern der Immunglobulinklasse IgG gegen SEQ ID NO1 nachgewiesen wird.

8. Verfahren oder Verwendung nach einem der Ansprüche 1 bis 7, wobei die Anwesenheit oder Abwesenheit eines Antikörpers aus der Gruppe der Immunglobulinklassen IgG, IgA und IgM gegen SEQ ID NO1 nachgewiesen wird.

9. Verfahren oder Verwendung nach einem der Ansprüche 1 bis 8, wobei die Anwesenheit oder Abwesenheit eines Antikörpers gegen SEQ ID NO1 für mindestens 1, 2, 3 oder 4 Wochen überwacht wird.

10. Verfahren oder Verwendung nach einem der Ansprüche 1 bis 9, wobei ein Verfahren ausgewählt aus der Gruppe umfassend Immundiffusionstechniken, immunelektrophoretische Techniken, Lichtstreuungsimmuntests, Agglutinationstechniken, Immuntests mit Markierung wie beispielsweise diejenigen aus der Gruppe umfassend radiomarkierte Immuntests, Enzym-Immuntests wie beispielsweise kolorimetrische Tests, Chemilumineszenz-Immuntests und Immunfluoreszenztechniken verwendet wird, um den Antikörper nachzuweisen.

11. Verfahren oder Verwendung nach einem der Ansprüche 1 bis 10, wobei ein Verfahren aus der Gruppe umfassend einen kompetitiven Test, einen capture bridge-Test, einen immunometrischen Test, einen klassenspezifischen sekundären Antikörper auf der festen Phase, einen class capture-Test und einen ELISA verwendet wird, um den Antikörper nachzuweisen.

12. Verfahren oder Verwendung nach einem der Ansprüche 1 bis 11, wobei die Probe eine Blutprobe aus der Gruppe umfassend Serum, Plasma und Vollblut ist.

13. Verfahren oder Verwendung nach einem der Ansprüche 1 bis 12, wobei eine Impfung durch die Bildung einer nachweisbaren Konzentration an Antikörpern erkannt werden kann.

14. Verfahren oder Verwendung nach Anspruch 13, wobei die nachweisbare Konzentration an Antikörpern Antikörper der Immunglobulinklassen IgA, IgM und IgG gegen SEQ ID NO1 umfasst.

15. Verfahren oder Verwendung nach einem der Ansprüche 1 bis 14, wobei dem Subjekt zwei oder drei oder mehr Impfdosen verabreicht wurden.

16. Verfahren oder Verwendung nach einem der Ansprüche 1 bis 15, wobei ein Mittel verwendet wird, das an den Antikörper der Immunglobulinklasse IgA bindet, das (a) ein sekundärer Antikörper ist, der an menschliche Antikörper der Immunglobulinklasse IgA bindet, oder (b) ein markiertes Polypeptid ist, das SEQ ID NO1 oder eine Variante davon umfasst, die an einen Antikörper aus der Gruppe umfassend die Immunglobulinklassen IgG, IgA und IgM gegen SEQ ID NO1 bindet,
wobei
a. das Polypeptid die Volllängensequenz von SEQ ID NO1 oder eine Variante von SEQ ID NO1 umfasst, die eine Sequenzidentität zu SEQ ID NO1 von mindestens 80, 85, 90, 92, 94, 95, 96, 97, 98, 99 % aufweist, optional fusioniert mit einem oder mehr als einem künstlichen Linker, einer oder mehr als einer Affinitätsmarkierung und/oder einem oder mehr als einem anderen Antigen, oder
b. die Variante eine verkürzte SEQ ID NO1 ist, d.h. weniger als die Volllängensequenz von SEQ ID NO1, wobei die Verkürzung am N-Terminus, C-Terminus oder an beiden vorliegt, optional fusioniert mit einem oder mehr als einem künstlichen Linker und/oder Affinitätstag, und
i. ein Fragment von SEQ ID NO1 umfasst, das mindestens 25, 50, 75, 100, 150, 200, 250, 300, 400, 500 oder 600 aufeinanderfolgende Aminosäuren von SEQ ID NO1 umfasst und/oder
ii. ein Fragment von SEQ ID NO1 umfasst, das mindestens 200 aufeinanderfolgende Aminosäuren mit einer Sequenzidentität zum Referenzfragment von SEQ ID NO1 von mindestens 80, 85, 90, 92, 94, 95, 96, 97, 98 oder 99 % umfasst,
und wobei die Variante die Fähigkeit hat, an einen Antikörper gegen SEQ ID NO1 aus einer Probe eines Patienten zu binden, der an SARS-CoV-2 leidet.

## Revendications

1. Procédé pour distinguer un sujet sain immunisé, qui est un sujet vacciné, d'un sujet sain non immunisé comprenant l'étape de détermination dans un échantillon de sang de la présence ou de l'absence d'un anticorps de classe IgA dirigé contre la SEQ ID NO: 1, l'échantillon comprenant un ensemble d'anticorps représentatifs du sujet, et l'échantillon de sang provenant d'un sujet humain.

2. Procédé pour tester l'efficacité d'un vaccin, comprenant l'étape de détermination dans un échantillon de sang de la présence ou de l'absence d'un anticorps de classe IgA dirigé contre la SEQ ID NO: 1, l'échantillon comprenant un ensemble d'anticorps représentatifs du sujet, et l'échantillon de sang provenant d'un sujet humain.

3. Utilisation d'un kit pour distinguer un sujet immunisé, qui est un sujet vacciné, d'un sujet non immunisé, le kit comprenant un support revêtu par un polypeptide comprenant la SEQ ID NO: 1 ou un variant correspondant et un moyen de détection d'un anticorps spécifiquement capturé
a. le polypeptide comprenant la séquence de pleine longueur de la SEQ ID NO: 1 ou un variant de la SEQ ID NO: 1, qui possède une identité de séquence avec la SEQ ID NO: 1 d'au moins 80, 85, 90, 92, 94, 95, 96, 97, 98, 99 %, éventuellement fusionné à un(e) ou plusieurs lieurs artificiels, étiquettes d'affinités et/ou d'autres antigènes, ou
b. le variant étant une SEQ ID NO: 1 tronquée, c'est-à-dire moins que la séquence de pleine longueur de la SEQ ID NO: 1, la troncature étant au niveau de la terminaison N, de la terminaison C ou des deux, éventuellement fusionné à un(e) ou plusieurs lieurs artificiels et/ou étiquettes d'affinités, et
i. comprenant un fragment de la SEQ ID NO: 1 comprenant au moins 25, 50, 75, 100, 150, 200, 250, 300, 400, 500 ou 600 acides aminés successifs de la SEQ ID NO: 1 et/ou
ii. comprenant un fragment de la SEQ ID NO: 1 comprenant au moins 200 acides aminés successifs avec une identité de séquence par rapport au fragment de référence de la SEQ ID NO: 1 d'au moins 80, 85, 90, 92, 94,95, 96, 97, 98 ou 99 %,
et le variant ayant la capacité de se lier à un anticorps dirigé contre la SEQ ID NO: 1 d'un échantillon d'un patient souffrant d'une infection par SARS-CoV-2,
comprenant l'étape de détermination dans un échantillon de sang de la présence ou de l'absence d'un anticorps de classe IgA dirigé contre la SEQ ID NO: 1, l'échantillon comprenant un ensemble d'anticorps représentatifs du sujet.

4. Procédé selon l'une quelconque des revendications 1 ou 3, un sujet sain non immunisé, un sujet sain immunisé et un sujet infecté étant distingués.

5. Procédé selon l'une quelconque des revendications 1 à 2 ou 4,
un support revêtu par un polypeptide comprenant la SEQ ID NO: 1 ou un variant correspondant étant mis en contact avec l'échantillon dans des conditions permettant une liaison de quelconques anticorps dirigés contre la SEQ ID NO: 1, et un anticorps ou un moyen secondaire de liaison à l'anticorps de classe IgA et portant un marqueur détectable étant mis en contact avec le support dans des conditions permettant la formation d'un complexe entre un quelconque anticorps lié et l'anticorps ou le moyen secondaire,
a. le polypeptide comprenant la séquence de pleine longueur de la SEQ ID NO: 1 ou un variant de la SEQ ID NO: 1, qui possède une identité de séquence avec la SEQ ID NO: 1 d'au moins 80, 85, 90, 92, 94, 95, 96, 97, 98, 99 %, éventuellement fusionné(e) à un(e) ou plusieurs lieurs artificiels, étiquettes d'affinités et/ou d'autres antigènes, ou
b. le variant étant une SEQ ID NO: 1 tronquée, c'est-à-dire moins que la séquence de pleine longueur de la SEQ ID NO: 1, la troncature étant au niveau de la terminaison N, de la terminaison C ou des deux, éventuellement fusionnée à un(e) ou plusieurs lieurs artificiels et/ou étiquettes d'affinités, et
i. comprenant un fragment de la SEQ ID NO: 1 comprenant au moins 25, 50, 75, 100, 150, 200, 250, 300, 400, 500 ou 600 acides aminés successifs de la SEQ ID NO: 1 et/ou
ii. comprenant un fragment de la SEQ ID NO: 1 comprenant au moins 200 acides aminés successifs avec une identité de séquence par rapport au fragment de référence de la SEQ ID NO: 1 d'au moins 80, 85, 90, 92, 94, 95, 96, 97, 98 ou 99 %,
et le variant ayant la capacité de se lier à un anticorps dirigé contre la SEQ ID NO: 1 d'un échantillon d'un patient souffrant d'une infection par SARS-CoV-2.

6. Procédé ou utilisation selon l'une quelconque des revendications 1 à 5, le support étant choisi dans le groupe comprenant une bille, une bande de dosage, une plaque de micro-titrage, un micro-réseau, un blot, préférablement dans le groupe comprenant un western blot, un line blot et un dot blot, une surface de verre, une lame, une biopuce et une membrane, et étant le plus préférablement une plaque de micro-titrage ou un line blot.

7. Procédé ou utilisation selon l'une quelconque des revendications 1 à 6, l'anticorps de classe IgA dirigé contre la SEQ ID NO: 1 étant détecté avant l'émergence d'anticorps de classe IgG dirigés contre la SEQ ID NO: 1.

8. Procédé ou utilisation selon l'une quelconque des revendications 1 à 7, la présence ou l'absence d'un anticorps du groupe de classes comprenant IgG, IgA et IgM dirigé contre la SEQ ID NO: 1 étant détectée.

9. Procédé ou utilisation selon l'une quelconque des revendications 1 à 8, la présence ou l'absence d'un anticorps dirigé contre la SEQ ID NO: 1 étant surveillée pendant au moins 1, 2, 3 ou 4 semaines.

10. Procédé ou utilisation selon l'une quelconque des revendications 1 à 9, un procédé choisi dans le groupe comprenant les techniques d'immunodiffusion, les techniques immunoélectrophorétiques, les immunodosages par diffusion de la lumière, les techniques d'agglutination, les immunodosages marqués tels que ceux du groupe comprenant les immunodosages radiomarqués, les immunodosages enzymatiques tels que les dosages colorimétriques, les immunodosages par chimiluminescence et les techniques d'immunofluorescence, étant utilisé pour détecter l'anticorps.

11. Procédé ou utilisation selon l'une quelconque des revendications 1 à 10, un procédé du groupe comprenant un dosage compétitif, un dosage de pont de capture, un dosage immunométrique, un deuxième anticorps spécifique à une classe sur la phase solide, un dosage de capture de classe et un dosage ELISA étant utilisé pour détecter l'anticorps.

12. Procédé ou utilisation selon l'une quelconque des revendications 1 à 11, l'échantillon étant un échantillon de sang du groupe comprenant le sérum, le plasma et le sang entier.

13. Procédé ou utilisation selon l'une quelconque des revendications 1 à 12, une vaccination pouvant être reconnue par la production d'un taux détectable d'anticorps.

14. Procédé ou utilisation selon la revendication 13, le taux détectable d'anticorps comprenant des anticorps de classe IgA, IgM et IgG dirigés contre la SEQ ID NO: 1.

15. Procédé ou utilisation selon l'une quelconque des revendications 1 à 14, deux ou trois injections de vaccins ayant été administrées au sujet.

16. Procédé ou utilisation selon l'une quelconque des revendications 1 à 15, un moyen de liaison à l'anticorps de classe IgA étant utilisé qui est (a) un anticorps secondaire se liant à des anticorps de classe IgA humains ou (b) un polypeptide marqué qui comprend la SEQ ID NO: 1 ou un variant correspondant qui se lie à un anticorps du groupe de classes comprenant IgG, IgA et IgM dirigé contre la SEQ ID NO: 1,
a. le polypeptide comprenant la séquence de pleine longueur de la SEQ ID NO: 1 ou un variant de la SEQ ID NO: 1, qui possède une identité de séquence avec la SEQ ID NO: 1 d'au moins 80, 85, 90, 92, 94, 95, 96, 97, 98, 99 %, éventuellement fusionné(e) à un(e) ou plusieurs lieurs artificiels, étiquettes d'affinités et/ou d'autres antigènes, ou
b. le variant étant une SEQ ID NO: 1 tronquée, c'est-à-dire moins que la séquence de pleine longueur de la SEQ ID NO: 1, la troncature étant au niveau de la terminaison N, de la terminaison C ou des deux, éventuellement fusionnée à un(e) ou plusieurs lieurs artificiels et/ou étiquettes d'affinités, et
i. comprenant un fragment de la SEQ ID NO: 1 comprenant au moins 25, 50, 75, 100, 150, 200, 250, 300, 400, 500 ou 600 acides aminés successifs de la SEQ ID NO: 1 et/ou
ii. comprenant un fragment de la SEQ ID NO: 1 comprenant au moins 200 acides aminés successifs avec une identité de séquence par rapport au fragment de référence de la SEQ ID NO: 1 d'au moins 80, 85, 90, 92, 94, 95, 96, 97, 98 ou 99 %,
et le variant ayant la capacité de se lier à un anticorps dirigé contre la SEQ ID NO: 1 d'un échantillon d'un patient souffrant d'un SARS-CoV-2.
